(19) Europäisches Patentamt
European Patent Office
Office européen des brevets

(11) EP 0 691 325 B1

(12) **EUROPÄISCHE PATENTSCHRIFT**

(45) Veröffentlichungstag und Bekanntmachung des Hinweises auf die Patenterteilung:
**15.04.1998 Patentblatt 1998/16**

(51) Int. Cl.$^6$: **C07C 67/283**, C07C 69/013, C07C 69/96

(21) Anmeldenummer: **95109592.6**

(22) Anmeldetag: **21.06.1995**

(54) **Asymmetrische Hydrierung von Ketoisophoron-Derivaten**
Asymmetric hydrogenation of ketoisophorone derivatives
Hydrogénation asymmétrique de dérivés de la cétoisophorone

(84) Benannte Vertragsstaaten:
**AT BE CH DE DK ES FR GB IT LI NL**

(30) Priorität: **07.07.1994 CH 2173/94**

(43) Veröffentlichungstag der Anmeldung:
**10.01.1996 Patentblatt 1996/02**

(73) Patentinhaber:
**F. HOFFMANN-LA ROCHE AG**
**4002 Basel (CH)**

(72) Erfinder:
- **Broger, Emil Albin**
  **CH-4312 Magden (CH)**
- **Crameri, Yvo**
  **CH-4104 Oberwil (CH)**
- **Schmid, Rudolf**
  **CH-4144 Arlesheim (CH)**
- **Siegfried, Theodor**
  **CH-4125 Riehen (CH)**

(74) Vertreter:
**Kjellsaa-Berger, Hanny, Dr. et al**
**F.Hoffmann-La Roche AG**
**Patent Department (PLP),**
**124 Grenzacherstrasse**
**4070 Basel (CH)**

(56) Entgegenhaltungen:
- **IWAO OJIMA 'CATALYTIC ASYMMETRIC SYNTHESIS' 1993 , VCH PUBLISHERS INC. , NEW YORK. US * Seite 1 - Seite 6 * * Seite 11 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 691 325 B1

Printed by Xerox (UK) Business Services
2.16.1/3.4

## Beschreibung

Die vorliegende Erfindung betrifft ein neues Verfahren zur enantioselektiven Hydrierung von Ketoisophoron-Derivaten und somit zur Herstellung von optisch aktiven Verbindungen der Formel

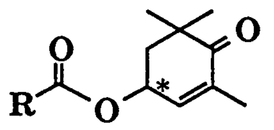

I

worin R niederes Alkyl, niederes Alkoxy, Phenyl, Benzyl oder $-NR^1_2$ , $R^1$ niederes Alkyl, Phenyl, Benzyl oder Wasserstoff und * ein optisch aktives Zentrum bedeutet.

Die Verbindungen der Formel I, welche durch Hydrolyse in Phorenol (**1**) und in einem weiteren Reaktionsschritt in optisch aktives Actinol (**2**) übergeführt werden können, sind wichtige Zwischenprodukte für die Herstellung von 3-Hydroxy-Carotenoiden, insbesondere für die Zeaxanthin-Herstellung (Helv. Chim. Acta 63 (1980), 1451). Es wurde bisher verschiedentlich versucht, das als Edukt für die Synthese von Actinol (**2**) verwendete Ketoisophoron (**3**) direkt enantioselektiv zu hydrieren. Dabei wurde sowohl mit Ruthenium- als auch mit Rhodiumkatalysatoren das Diketon (**6**) als Hauptprodukt erhalten, die optischen Ausbeuten waren jedoch nur gering. Es wurde auch versucht, durch Derivatisierung des Ketoisophorons (**3**) ein für die Hydrierung geeigneteres Substrat herzustellen. So ergab beispielsweise die asymmetrische Hydrierung des cyclischen Acetals (**5**) und anschliessende Hydrolyse das Diketon (**6**) mit nur 10% enantiomeren Ueberschuss (ee), während bei der asymmetrischen Hydrierung des Methylenolethers (**4**) das Diketon (**6**) mit 50% ee erhalten wurde (Brunner et al. J. Organomet. Chem. 456 (1993), 71 und dort zitierte Literatur).

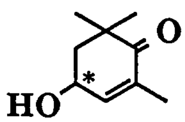

(1)

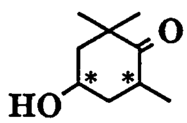

(2)

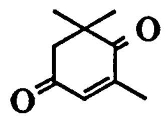

(3)

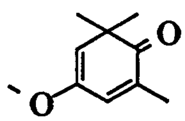

(4)

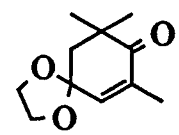

(5)

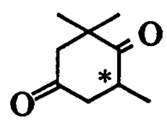

(6)

Einen technisch interessanten Zugang zu Actinol (**2**) durch asymmetrische Hydrierung ermöglicht nun die vorliegende Erfindung. Es wurde gefunden, dass die enantioselektive Hydrierung von Acyl-Derivaten des Ketoisophorons in Gegenwart eines kationischen Rhodiumkomplexes mit chiralen Diphosptunliganden in geeigneten Lösungsmittel in hohen optischen Ausbeuten zum entsprechenden Acyl-Derivat von Phorenol führt.

Die Erfindung betrifft somit ein Verfahren zur Herstellung von Verbindungen der allgemeinen Formel

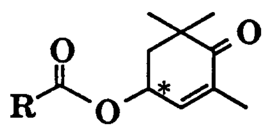

I

worin R niederes Alkyl, niederes Alkoxy, Phenyl, Benzyl oder $-NR^1_2$ , $R^1$ niederes Alkyl, Phenyl, Benzyl oder Wasserstoff und * ein optisch aktives Zentrum bedeutet,
dadurch gekennzeichnet, dass man ein Enol-Derivat von Ketoisophoron der allgemeinen Formel

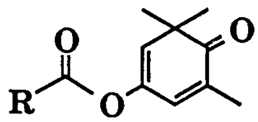
II

worin R die oben angegebene Bedeutung hat,
in Gegenwart eines Rhodiumkomplexes eines optisch aktiven Diphosphinliganden asymmetrisch hydriert.

Als Katalysator werden erfindungsgemäss kationische Rhodiumkomplexe der Formel

$$[Rh(Y)L_n]^+A^- \qquad III$$

verwendet, worin

L ein neutraler Ligand,
n 0, 1 oder 2,
$A^-$ ein Anion wie $BF_4^-$, $CF_3SO_3^-$, $PF_6^-$, $ClO_4^-$, $B(C_6H_5)_4^-$, und
Y ein chirales Diphosphin bedeutet.

Insbesondere eignen sich kationische Komplexe der Formel III, worin das chirale Diphosphin (Y) eine Verbindung der Formeln

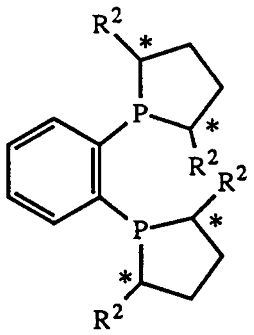

IV

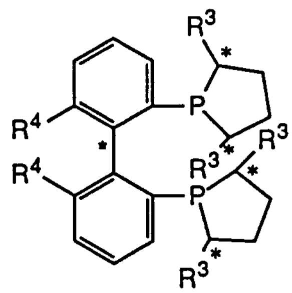

V

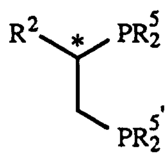

VI

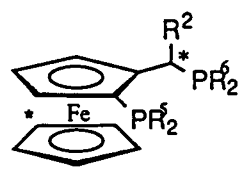

VII

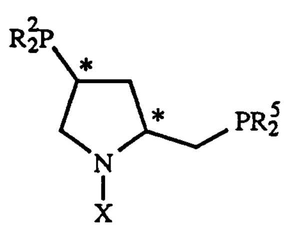

VIII

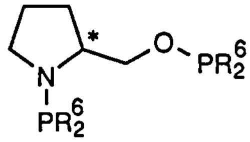

IX

oder
darstellt, worin

X -COR$^6$, COOR$^6$, CONR$^3_2$ , SO$_2$R$^6$ oder POR$^5_2$ ,
$R^2$ Cycloalkyl oder Alkyl,
$R^3$ Wasserstoff, Cycloalkyl oder Alkyl,
$R^4$ niederes Alkyl oder niederes Alkoxy,
$R^5$ und $R^{5'}$ Aryl oder Heteroaryl, und
$R^6$ Aryl, Heteroaryl, Cycloalkyl oder Alkyl bedeutet,

wobei die Reste $R^5$ und $R^{5'}$ gleich oder verschieden sein können.

Der Ausdruck "niederes Alkyl" bedeutet im Rahmen der vorliegenden Erfindung geradkettige oder verzweigte Alkylgruppen mit 1 bis 4 Kohlenstoffatomen wie Methyl, Ethyl, Propyl, Isopropyl, n-Butyl, Isobutyl und tert.Butyl. Der Ausdruck "niederes Alkoxy" bedeutet Gruppen, worin der Alkylrest die vorhergehende Bedeutung hat.

Der Ausdruck "neutraler Ligand" bedeutet im Rahmen der vorliegenden Erfindung einen leicht austauschbaren Liganden, wie Olefine, z.B. Ethylen, Propylen, Cycloocten, 1,5-Hexadien, Norbornadien, 1,5-Cyclooctadien und dergleichen oder Nitrile, wie Acetonitril, Benzonitril. Der Ligand kann bei der Hydrierung auch ausgetauscht werden. Falls mehr als ein solcher Ligand vorhanden ist, können diese auch voneinander verschieden sein.

Sowohl der im Zusammenhang mit den Verbindungen der Formeln I und II genannte Phenylrest als auch der Benzylrest können im Rahmen der vorliegenden Erfindung gegebenenfalls in ortho-, meta- oder para-Stellung niedere Alkyl- oder niedere Alkoxygruppen aufweisen.

Der im Zusammenhang mit den Formeln IV-IX verwendete Ausdruck Alkyl bedeutet geradkettige oder verzweigte Alkylreste mit 1 bis 7 Kohlenstoffatomen. Cycloalkyl bedeutet in diesem Rahmen Cyclopropyl, Cyclobutyl, Cyclopentyl, Cyclohexyl oder Cycloheptyl.

Der im Zusammenhang mit den Verbindungen der Formeln VI, VII, VIII und IX verwendete Ausdruck "Aryl" bedeutet im Rahmen der vorliegenden Erfindung Phenyl, welches gegebenenfalls in ortho-, meta- oder para-Stellung niederes Alkyl oder niederes Alkoxy, vorzugsweise Methyl oder Methoxy, oder auch Di-niederes Alkylamino, vorzugsweise Dimethylamino aufweisen kann.

Der Ausdruck "Heteroaryl" steht im Rahmen der vorliegenden Erfindung für einen Rest der Formel

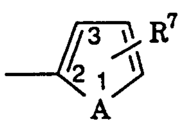
(a)

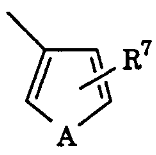
(b)

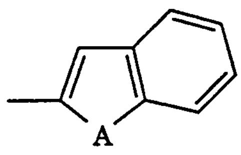
(c)

oder

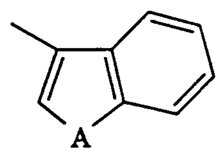
(d)

In den Resten der Formeln (a) bis (d) wiederum bedeutet A Sauerstoff, Schwefel oder -NR$^8$. Der Substituent $R^7$ bedeutet hier Wasserstoff, niederes Alkyl, insbesondere Methyl, oder niederes Alkoxy, insbesondere Methoxy, und $R^8$ steht für niederes Alkyl, vorzugsweise Methyl.

Für das erfindungsgemässe Verfahren eignen sich kationische Rhodium-komplexe der Formel III mit den chiralen Diphosphinliganden der Formeln IV-IX, insbesondere mit Diphosphinliganden der Formeln IV, VI und VII.

Sehr hohe Selektivitäten werden im erfindungsgemässen Verfahren bei der Verwendung von kationischen Rhodiumkomplexen der Formel III mit chiralen Diphosphinliganden der Formeln IV und VII erzielt. Eine besonders hohe Selektivität bei einem praktisch quantitativen Umsatz des Substrates II und hohen optischen Ausbeuten kann insbesondere mit Rhodiumkomplexen mit chiralen Diphosphinliganden der Formel IV, worin $R^2$ Methyl oder Ethyl bedeutet, erreicht werden.

Die Liganden der Formeln IV und V sind bekannte Verbindungen und können beispielsweise wie in US Pat. Nr. 5 171 892 beschrieben, hergestellt werden. Die chiralen Liganden der Formel VI sind ebenfalls bekannte Verbindungen und können beispielsweise nach dem in EP-A-031 877 beschriebenen Verfahren hergestellt werden. Die Liganden der Formel VII können analog zu EP-A-564 406 erhalten werden, während ein mögliches Verfahren zur Herstellung der Liganden der Formel VIII in EP-A-251 164 beschrieben wird. Die Herstellung von Liganden der Formel IX kann beispielsweise nach der in Tetrahedron: *Asymmetrie* 1993, Vol. 4, 2279, angegebenen Methode erfolgen.

Die enantioselektive Hydrierung von Verbindungen der Formel II kann in einem gegenüber dieser Verbindung inerten, aprotischen Medium wie einem Ester, Ether oder einem Kohlenwasserstoff durchgeführt werden. Als geeignete Lösungsmittel können insbesondere Ester wie Ethylacetat, cyclische Ether wie beispielsweise Tetrahydrofuran oder Dioxan, aromatische Kohlenwasserstoffe wie Benzol oder Toluol oder auch Gemische hiervon verwendet werden.

Das Verhältnis zwischen Rhodium und dem Liganden Y liegt zweckmässig zwischen etwa 0,05 und 5 Mol, vorzugsweise zwischen 0,5 und 2 Mol Rhodium per Mol Ligand. Das molare Verhältnis zwischen Rhodium im Komplex der Formel III und der zu hydrierenden Verbindung der Formel II liegt zweckmässig zwischen etwa 0,001 und etwa 5 Mol%, vorzugsweise zwischen etwa 0,002 und etwa 0,2 Mol%; d.h. das Substrat/Katalysator Verhältnis (S/C) beträgt 100'000 bis etwa 20, insbesondere etwa 50'000 bis etwa 500. Die enantioselektive Hydrierung von Verbindungen der Formel II unter Verwendung eines Komplexes der Formel III kann bei Temperaturen von etwa 10°C bis etwa 120°C, vorzugsweise bei etwa 10°C bis etwa 60°C, erfolgen. Die Hydrierung erfolgt unter Druck, zweckmassig unter einem Druck von etwa 1 bis 150 bar, vorzugsweise von 5 bis 60 bar.

Eine bevorzugte Ausführung des erfindungsgemässen Verfahrens betrifft die Herstellung der (R)- oder (S)-Enantiomeren der Verbindung der Formel

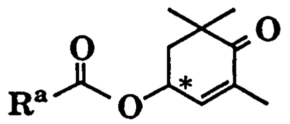

I-a

worin $R^a$ niederes Alkyl, insbesondere Methyl bedeutet,
durch asymmetrische Hydrierung der entsprechenden Verbindung der Formel

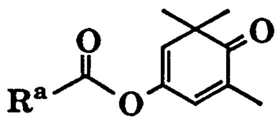

II-a

worin $R^a$ die oben angegebene Bedeutung hat.

Die Rhodiumkomplexe der allgemeinen Formel III können in an sich bekannter Weise hergestellt werden, z.B. nach dem in EP-A-0 574 783 beschriebenen Verfahren, indem man eine Verbindung der Formeln IV bis IX mit einer Verbindung, welche Rhodium abgeben kann, in einem geeigneten, inerten Lösungsmittel umsetzt.

Die folgenden Beispiele dienen zur Erläuterung der Erfindung und stellen keinerlei Einschränkungen dar.

GC = Kapillar-Gaschromatographie
o.p. = optische Reinheit
ee = Enantiomerer Ueberschuss
Essigester = Ethylacetat
(R,R)-Et-DuPHOS = 1,2-Bis[(2R,5R)-2,5-diethylphospholano]benzol
(S,S)-Et-DuPHOS = 1,2-Bis[(2S,5S)-2,5-diethylphospholano]benzol
(R,R)-Me-DuPHOS = 1,2-Bis[(2R,5R)-2,5-dimethylphospholano]benzol
(S,S)-Me-DuPHOS = 1,2-Bis[(2S,5S)-2,5-dimethylphospholano]benzol
(R)-PROPHOS = (R)-1,2-Bis(diphenylphosphino)propan
(S,S)-BCPM = (2S,4S)-1-tert-Butoxycarbonyl-4-dicyclohexylphosphino-2-diphenylphosphinomethylpyrrolidin
(R,S)-PPF-$PCy_2$ = {(R)-1-[(S)-2-(Diphenylphosphino)ferrocenyl]}ethyldicyclohexylphosphin
(R,S)-PPF-$P(tBu)_2$ = {(R)-1-[(S)-2-(Diphenylphosphino)ferrocenyl]}ethyl-di-tert.-butylphosphin
(R,S)-PPF-$P(o\text{-}An)_2$ = {(R)-1-[(S)-2-(Diphenylphosphino)ferrocenyl]}ethyl-di-(o-methoxyphenyl)phosphin
(S,S,S)-MePHOS-MeOBIPHEP = (2S,5S)-1-{(S)-2'-[(2S,5S)-2,5-Dimethylphospholan-1-yl]-6,6'-dimethoxy-biphenyl-2-yl}-2,5-dimethyl-phospholan
(S)-MeOBIPHEPHOS = 1-{(S)-2'-(Phospholan-1-yl)-6,6'-dimethoxybiphenyl-2-yl}-phospholan
(S,S,S)-MePHOS-MeOBIPHEP = (2S,5S)-1-{(S)-2'-[(2S,5S)-2,5-Dimethylphospholan-1-yl]-6,6'-dimethoxy-biphenyl-2-yl}-2,5-dimethyl-phospholan
(S,S,R)-MePHOS-MeOBIPHEP = (2S,5S)-1-{(R)-2'-[(2S,5S)-2,5-Dimethylphospholan-1-yl]-6,6'-dimethoxy-biphenyl-2-yl}-2,5-dimethyl-phospholan
$Cy_4$-5-OxoProNOP= (R)-1-Dicyclohexylphosphanyl-5-(dicyclohexylphosphanyoxymethyl)-pyrrolidin-2-one

Beispiel 1

In einer Glove-Box ($O_2$-Gehalt < 1ppm) wurden 10.5 mg (0.026 mMol) Bis-(1,5-Cyclooctadien)rhodium(I)tetrafluoroborat und 9.4 mg (0.026 mMol) (R,R)-Et-DuPHOS in einen 20 ml Messkolben gegeben und mit ca. 20 ml Essigester auf die Marke aufgefüllt. Die erhaltene orange Katalysatorlösung wurde 10 Min. bei 22°C gerührt.

In der Glove-Box wurden dann 25.0 g (128.7 mMol) 4-Acetoxy-2,6,6-trimethylcyclohexa-2,4-dien-1-on (hergestellt gemäss Helv. Chim. Acta 1989, **72**, 365 und zusätzlich kristallisiert aus n-Hexan, Smp. 44-45°C) und 20 ml Essigester in einen 185 ml-Stahlautoklaven vorgelegt. Zu dieser Suspension wurden 10 ml der oben hergestellten Katalysatorlösung (S/C 10'000) gegeben und der Autoklav verschlossen. Die Hydrierung wurde bei 20°C, einem konstanten Druck von 10 bar und intensivem Rühren durchgeführt.

Nach 21 Stunden betrug der Umsatz 100%. Die Hydrierlösung bestand aus einem Gemisch von 92.8 GC-Fl% 4-Acetoxy-2,6,6-trimethyl-2-cyclohexen-1-on und 7.2% 4-Acetoxy-2,6,6-trimethyl-4-cyclohexen-1-on.

Zur ee-Bestimmung wurde eine ca. 20 mg Produkt enthaltende Probe der Hydrierlösung bei 50°C/17 mbar eingedampft, der Rückstand in 1 ml Methanol gelöst und mit 20 mg Natriummethylat, unter Erwärmen auf 50°C während 30 Min., zum Gemisch von 92% (4S)-4-Hydroxy-2,6,6-trimethyl-2-cyclohexen-1-on (98.5% ee) und 8% 2,2,6-Trimethylcyclohexan-1,4-dion (rac.) umgesetzt. Nach Ansäuern mit ca. 60 mg Essigsäure erfolgte die eeBestimmung durch Gaschromatographie an einer chiralen Phase (permethyliertes β-Cyclodextrin gemischt mit OV-61).

Zur Aufarbeitung wurde die aus dem Autoklaven gespülte Hydrierlösung bei 45°C/20 mbar eingedampft. Der Rückstand, 25.2 g dunkelgelbes Oel wurde in 125 ml Methanol und 25 ml Wasser gelöst und bei 20°C 25.5 ml 5N wässerige Natronlauge zugetropft. Nach einstündigem Rühren bei 20°C wurde das Methanol bei 50°C/23 mbar abgedampft. Die wässerige Phase wurde mit Diethylether extrahiert. Nach Trocknen und Eindampfen der organischen Phase wurden 18.9 g oranges Oel erhalten, welches an 500 g Kieselgel mit n-Hexan/Ether 3/1 chromatographiert wurde. Man erhielt 17.8 g (90%) (4S)-4-Hydroxy-2,6,6-trimethyl-2-cyclohexen-1-on als hellgelbes Oel; chem. Reinheit 99.2 GC-Fl%; 98.5% ee; $[\alpha]_D$ = -48.8° (c=1, EtOH).

Beispiel 2

a) Synthese von 4-tert.-Butyloxycarbonyloxy-2,6,6-trimethylcyclohexa-2,4-dienon:

Zu einer bei 24°C gerührten Suspension von 20.0 g (144.7 mMol) $K_2CO_3$, 20 ml Tetrahydrofuran, 1.1 g (4.2 mMol) 18-Crown-6 und 15.2 g (100 mMol) Ketoisophoron, wurde eine Lösung von 23.2 g (106.3 mMol) Di-tert.-butyldicarbonat in 25 ml Tetrahydrofuran zugetropft. Die Suspension wurde 5 Stunden bei 60°C gerührt, dann das braune Reaktionsgemisch filtriert und schliesslich das Lösungsmittel abgedampft. Der braune Rückstand (26.6 g) wurde in 200 ml Dichlormethan gelöst, und die Lösung mit gesättigter $NaHCO_3$-Lösung und mit Wasser extrahiert. Die organische Phase wurde über $Na_2SO_4$ getrocknet, filtriert und eingedampft. Der braune Rückstand (22.6 g) wurde über 150 g Kieselgel chromatographiert (n-Hexan/Ether 2/1). Man erhielt 20.4 g (81 %) 4-tert.-Butyloxycarbonyloxy-2,6,6-trimethylcyclohexa-2,4-dienon als gelbes Oel; Anal. ber. für $C_{14}H_{20}O_4$ (252.31): C 66.65, H 7.99; gef.: C 66.46, H 8.08. $^1$H-NMR (250 MHz, $CDCl_3$): 1,25 (s, 2 $CH_3$-C(6)); 1.54 (s, $(CH_3)_3C$-O); 1.91 (s, $CH_3$-C(2); 5.88-5.89 (d, H-C(5)); 6.71-6.74 (m, H-C(5)).

b) Hydrierung von 4-tert.-Butyloxycarbonyloxy-2,6,6-trimethylcyclohexa-2,4-dienon:

In einer Glove-Box ($O_2$-Gehalt < 1ppm) wurden 40.3 mg (0.099 mMol) Bis(1,5-Cyclooctadien)rhodium(I)tetrafluoroborat und 35.9 mg (0.099 mMol) (R,R)-Et-DuPHOS in einen 20 ml Messkolben gegeben und mit ca. 20 ml Essigester auf die Marke aufgefüllt. Die erhaltene orange Katalysatorlösung wurde 10 Min. bei 22°C gerührt.

In der Glove-Box wurden dann 2.5 g (9.9 mMol) 4-tert.-Butyloxycarbonyloxy-2,6,6-trimethylcyclohexa-2,4-dienon und 47 ml Essigester in einen 185 ml-Stahlautoklaven vorgelegt, 20 ml der oben hergestellten Katalysatorlösung (S/C 100) zugegeben und der Autoklav verschlossen. Die Hydrierung wurde bei 20°C, einem konstanten Druck von 10 bar und intensivem Rühren durchgeführt.

Nach 5 Stunden betrug der Umsatz 100%. Die Hydrierlösung bestand aus einem Gemisch von 98 GC-Fl% 4-tert.-Butyloxycarbonyloxy-2,6,6-trimethyl-2-cyclohexen-1-on und 2% 4-tert.-Butyloxycarbonyloxy-2,6,6-trimethyl-4-cyclohexen-1-on.

Zur ee-Bestimmung wurde eine ca. 20 mg Produkt enthaltende Probe der Hydrierlösung bei 50°C/17 mbar eingedampft, der Rückstand in 1 ml Methanol gelöst und mit 20 mg Natriummethylat, unter Erwärmen auf 50°C während 30 Min., zum Gemisch von 98% (4S)-4-Hydroxy-2,6,6-trimethyl-2-cyclohexen-1-on (87.3% ee) und 2% 2,2,6-Trimethylcyclohexan-1,4-dion (rac.) umgesetzt. Nach Ansäuern mit ca. 60 mg Essigsäure erfolgte die ee-Bestimmung durch Gaschromatographie an einer chiralen Phase (permethyliertes β-Cyclodextrin gemischt mit OV-61).

Beispiel 3

a) Synthese von 4-Benzoyloxy 2,6,6-trimethylcyclohexa-2,4-dien-1-on:

Ein Gemisch aus 91.3 g (0.6 Mol) Ketoisophoron, 600 g (2.65 Mol) Benzoesäureanhydrid, 251 ml (1.8 Mol) Triethylamin und 3.66 g (0.03 Mol) 4-(Dimethylamino)pyridin wurde bei 65°C unter Argon 18 Stunden gerührt und mit 1 l Ether verdünnt. Bei 0-10°C wurden 260 ml Ethylendiamin unter Kühlen zugetropft. Der Niederschlag wurde abfiltriert und mit 1.5 l Ether gewaschen. Das vereinigte Filtrat wurde mit gesättigter $NaHCO_3$-Lösung (1 l), dann mit gesättigter Kochsalzlösung extrahiert, über $Na_2SO_4$ getrocknet und eingedampft. Das Rohprodukt (137.8 g), ein brauner kristalliner Rückstand wurde über Kieselgel chromatographiert (n-Hexan/Ether 5/1). Man erhielt 117.4 g (76.3%) 4-Benzoyloxy 2,6,6-trimethylcyclohexa-2,4-dien-1-on als gelbes Kristallisat. Umkristallisation aus tert.-Butyl-methylether/n-Hexan (1/1) lieferte reines Benzoat, Smp. 64-66°C.

b) Hydrierung von 4-Benzoyloxy 2,6,6-trimethylcyclohexa-2,4-dien-1-on:

In einer Glove-Box ($O_2$-Gehalt < 1ppm) wurden 19.8 mg (0.049 mMol) Bis(1,5-Cyclooctadien)rhodium(I)tetrafluoroborat und 17.7 mg (0.049 mMol) (S,S)-Et-DuPHOS in einen 20 ml Messkolben gegeben und mit ca. 20 ml Essigester auf die Marke aufgefüllt. Die erhaltene orange Katalysatorlösung wurde 10 Min. bei 22°C gerührt.

In der Glove-Box wurden dann 2.5 g (9.75 mMol) 4-Benzoyloxy-2,6,6-trimethylcyclohexa-2,4-dien-1-on und 62.6 ml Essigester in einen 185 ml-Stahlautoklaven vorgelegt, 4 ml der oben hergestellten Katalysatorlösung (S/C 1000) zugegeben und der Autoklav verschlossen. Die Hydrierung wurde bei 20°C, einem konstanten Druck von 10 bar und intensivem Rühren durchgeführt.

Nach 43 Stunden betrug der Umsatz 100%. Die Hydrierlösung bestand aus einem Gemisch von 96 GC-Fl% 4-Benzoyloxy-2,6,6-trimethyl-2-cyclohexen-1-on und 4% 4-Benzoyloxy-2,6,6-trimethyl-4-cyclohexen-1-on. Zur ee-Bestimmung wurde eine ca. 20 mg Produkt enthaltende Probe der Hydrierlösung bei 50°C/17 mbar eingedampft, der Rückstand in 1 ml Methanol gelöst und mit 20 mg Natriummethylat, unter Erwärmen auf 50°C während 30 Min., zum Gemisch von 96% (4R)-4-Hydroxy-2,6,6-trimethyl-2-cyclohexen-1-on (91.2% ee) und 4% 2,2,6-Trimethyl-cyclohexan-1,4-dion umgesetzt. Nach Ansäuern mit ca. 60 mg Essigsäure erfolgte die ee-Bestimmung durch Gaschromatographie an einer chiralen Phase (permethyliertes β-Cyclodextrin gemischt mit OV-61).

Beispiele 4 - 22: siehe Tabelle 1.

Tabelle 1: Asymmetrische Hydrierung von ...etat in Gegenwart von ...h(P⌒P)]X [5)]

| Nr. | P⌒P | X | S/C mol/mol | Solvens | c % | p bar | T °C | % Ums. nach 20h | Selekt. % 1) | ee (S) % |
|---|---|---|---|---|---|---|---|---|---|---|
| 4 | (R,R)-Et-DuPHOS | $CF_3SO_3$ | 100 | Ethylacetat | 4 | 10 | 20 | 100 | 77 | 92.7 |
| 4a | (R,R)-Me-DuPHOS | $BF_4$ | 15000 | Methylacetat | 48 | 10 | 20 | 100 | 93 | 97.5 |
| 5 | (R,R)-Et-DuPHOS | $PF_6$ | 100 | Ethylacetat | 4 | 10 | 20 | 100 | 84 | 92.0 |
| 6 | (R,R)-Me-DuPHOS | $BF_4$ | 10000 | Ethylacetat | 48 | 10 | 20 | 100 | 95 | 98.5 |
| 7 | (R,R)-Et-DuPHOS | $CF_3SO_3$ | 200 | Ethylacetat | 4 | 5 | 20 | 99 | 78 | 90.7 |
| 8 | (R,R)-Et-DuPHOS | $BF_4$ | 10000 | Ethylacetat | 80 | 10 | 20 | 100 | 91 | 98.3 |
| 9 | (R,R)-Et-DuPHOS | $BF_4$ | 10000 | Ethylacetat | 24 | 50 | 20 | 99 | 92 | 96.4 |
| 10 | (R,R)-Et-DuPHOS | $BF_4$ | 10000 | Ethylacetat | 24 | 10 | 60 | 96 | 89 | 96.9 |
| 11 | (R,R)-Et-DuPHOS | $BF_4$ | 10000 | Ethylacetat | 24 | 50 | 60 | 94 | 91 | 97.1 |
| 12 | (R,R)-Et-DuPHOS | $BF_4$ | 10000 | Ethylacetat | 12 | 10 | 100 | 99 | 85 | 89.2 |
| 13 | (R,R)-Et-DuPHOS | $BF_4$ | 500 | Tetrahydrofuran | 12 | 10 | 20 | 100 | 83 | 97.5 |
| 14 | (R,R)-Et-DuPHOS | $BF_4$ | 500 | Toluol | 12 | 10 | 60 | 99 | 80 | 83.8 |
| 15 | (R,S)-PPF-$PCy_2$ | $BF_4$ | 100 | Ethylacetat | 8 | 10 | 20 | 100 | 22[2)] | 79.6 |
| 16 | (R,S)-PPF-$P(tBu)_2$ | $BF_4$ | 100 | Ethylacetat | 8 | 10 | 20 | 100 | 12[3)] | 94.2 |
| 16a | (R,S)-PPF-$P(tBu)_2$ | $BF_4$ | 5000 | Ethylacetat | 12 | 10 | 20 | 78 | 97 | 83.9 |
| 16b | (R,S)-PPF-$P(o\text{-}An)_2$ | $BF_4$ | 1000 | Ethylacetat | 12 | 10 | 20 | 97 | 61 | 86.3 |
| 17 | (S,S)-BCPM | $BF_4$ | 100 | Ethylacetat | 8 | 10 | 20 | 100 | 47[4)] | 89.8(R) |
| 18 | (R)-PROPHOS | $CF_3SO_3$ | 100 | Ethylacetat | 4 | 10 | 20 | 100 | 65 | 81.3 |
| 19 | (S,S,S)-MePHOS-MeOBIPHEP | $BF_4$ | 100 | Ethylacetat | 12 | 10 | 20 | 96 | 70 | 70.8 |
| 20 | (S,S,R)-MePHOS-MeOBIPHEP | $BF_4$ | 100 | Ethylacetat | 12 | 10 | 20 | 100 | 47 | 60.9 |
| 21 | (S)-MeOBIPHEPHOS | BF4 | 100 | Ethylacetat | 8 | 10 | 20 | 100 | 48 | 58.7 |
| 22 | (S)-$Cy_4$-oxoProNOP | $BF_4$ | 1000 | Ethylacetat | 12 | 10 | 20 | 100[6)] | 79 | 90.6 (R) |

1) Nebenprodukt: 4-Acetoxy-2,6,6-trimethyl-4-cyclohexen-1-on
2) Nebenprodukt: 64 GC-Fl% 4-Acetoxy-2,6,6-trimethyl-4-cyclohexan-1-on
3) Nebenprodukt: 77 GC-Fl% 4-Acetoxy-2,6,6-trimethyl-4-cyclohexan-1-on
4) Nebenprodukt: 65 GC-Fl% 4-Acetoxy-2,6,6-trimethyl-4-cyclohexan-1-on

5) Katalysator hergestellt in situ aus $[Rh(COD)_2]X$ + P⌒P analog zu Beispiel 1
6) Umsatz nach 6 Stunden

## Patentansprüche

1. Verfahren zur Herstellung von optisch aktiven Verbindungen der allgemeinen Formel

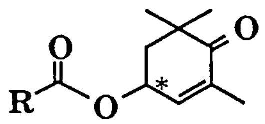

I

worin R niederes Alkyl, niederes Alkoxy, Phenyl, Benzyl oder $-NR^1_2$ , $R^1$ niederes Alkyl, Phenyl, Benzyl oder Wasserstoff und * ein optisch aktives Zentrum bedeutet,
dadurch gekennzeichnet, dass man ein Enol-Derivat von Ketoisophoron der allgemeinen Formel

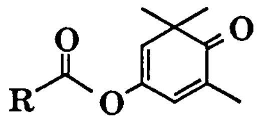

II

worin R die oben angegebene Bedeutung hat,
in Gegenwart eines Rhodiumkomplexes eines optisch aktiven Diphosphinliganden asymmetrisch hydriert.

2. Verfahren nach Anspruch 1, dadurch gekennzeichnet, dass man die asymmetrische Hydrierung durchführt in Gegenwart eines kationischen Rhodiumkomplexes der Formel

$$[Rh(Y)L_n]^+A^- \qquad III$$

worin

L ein neutraler Ligand,
n 0, 1 oder 2,
$A^-$ ein Anion wie $BF_4^-$, $CF_3SO_3^-$, $PF_6^-$, $ClO_4^-$, $B(C_6H_5)_4^-$, und
Y ein chirales Diphosphin bedeutet.

3. Verfahren nach Anspruch 2, dadurch gekennzeichnet, dass das chirale Diphosphin eine Verbindung der Formel

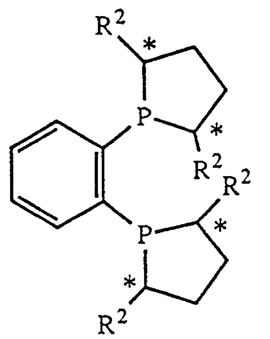

IV

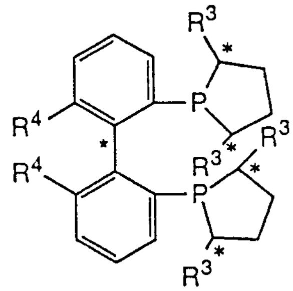

V

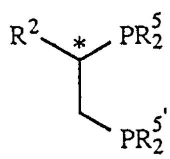

VI

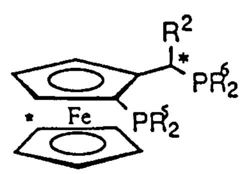

VII

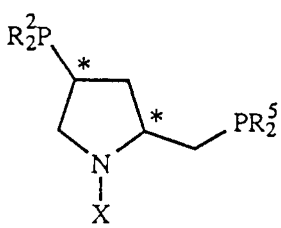

VIII

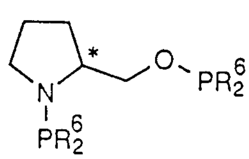

IX

oder

darstellt, worin

| | |
|---|---|
| X | $COR^6$, $COOR^6$, $CONR^3_2$ , $SO_2R^6$ oder $POR^5_2$ , |
| $R^2$ | Cycloalkyl oder Alkyl, |

| | |
|---|---|
| $R^3$ | Wasserstoff, Cycloalkyl oder Alkyl, |
| $R^4$ | niederes Alkyl oder niederes Alkoxy, |
| $R^5$ und $R^{5'}$ | Aryl oder Heteroaryl, und |
| $R^6$ | Aryl, Heteroaryl, Cycloalkyl oder Alkyl bedeutet, |

wobei $R^5$ und $R^{5'}$ gleich oder verschieden sein können.

**4.** Verfahren nach Anspruch 3, dadurch gekennzeichnet, dass das chirale Diphosphin eine Verbindung der Formeln IV oder VII darstellt.

**5.** Verfahren nach Anspruch 3, dadurch gekennzeichnet, dass das chirale Diphosphin eine Verbindung der Formel IV ist, worin $R^2$ Methyl oder Ethyl bedeutet.

## Claims

**1.** A process for the manufacture of optically active compounds of the general formula

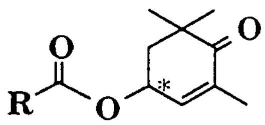

I

wherein R signifies lower alkyl, lower alkoxy, phenyl, benzyl or $-NR^1_2$, $R^1$ signifies lower alkyl, phenyl, benzyl or hydrogen and * signifies an optically active centre,
characterized by asymmetrically hydrogenating an enol derivative of ketoisophorone of the general formula

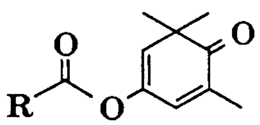

II

wherein R has the significance given above,
in the presence of a rhodium complex of an optically active diphosphine ligand.

**2.** A process according to claim 1, characterized in that the asymmetric hydrogenation is carried out in the presence of a cationic rhodium complex of the formula

$$[Rh(Y)L_n]^+A^- \qquad III$$

wherein

| | |
|---|---|
| L | signifies a neutral ligand, |
| n | signifies 0, 1 or 2, |
| $A^-$ | signifies an anion such as $BF_4^-$, $CF_3SO_3^-$, $PF_6^-$, $ClO_4^-$, $B(C_6H_5)_4^-$, and |
| Y | signifies a chiral diphosphine. |

**3.** A process according to claim 2, characterized in that the chiral diphosphine is a compound of the formula

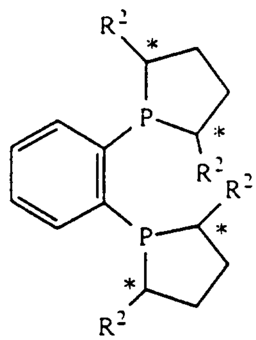

IV

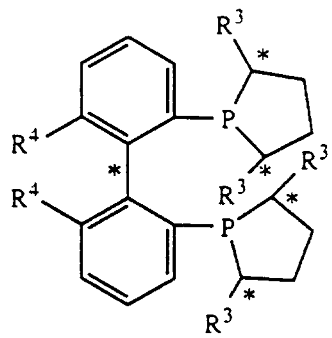

V

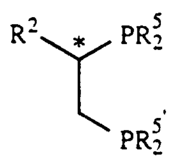

VI

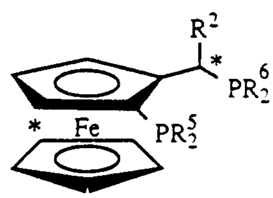

VII

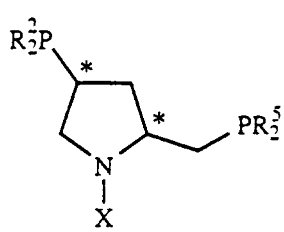

VIII

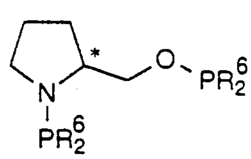

IX

wherein

| | |
|---|---|
| X | signifies $-COR^6$, $COOR^6$, $CONR^3_2$, $SO_2R^6$ or $POR^5_2$, |
| $R^2$ | signifies cycloalkyl or alkyl, |
| $R^3$ | signifies hydrogen, cycloalkyl or alkyl, |
| $R^4$ | signifies lower alkyl or lower alkoxy, |
| $R^5$ and $R^{5'}$ | signify aryl or heteroaryl and |
| $R^6$ | signifies aryl, heteroaryl, cycloalkyl or alkyl, |

whereby $R^5$ and $R^{5'}$ can be the same of different.

4. A process according to claim 3, characterized in that the chiral diphosphine is a compound of formula IV or VII.

5. A process according to claim 3, characterized in that the chiral diphosphine is a compound of formula IV in which $R^2$ signifies methyl or ethyl.

**Revendications**

1. Procédé pour la préparation des composés optiquement actifs de formule générale

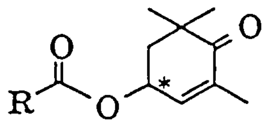

I

où R représente un alkyle inférieur, un alcoxy inférieur, le phényle, le benzyle ou $-NR_2^1$, $R^1$ représentant un alkyle inférieur, le phényle, le benzyle ou l'hydrogène et * un centre optiquement actif,

caractérisé en ce que l'on hydrogène asymétriquement un dérivé énolique de cétoisophorone de formule générale

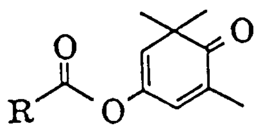

II

où R a la signification indiquée ci-dessus,

en présence d'un complexe du rhodium d'un ligand diphosphine optiquement actif.

2. Procédé selon la revendication 1, caractérisé en ce que l'on effectue l'hydrogénation asymétrique en présence d'un complexe cationique du rhodium de formule

$$[Rh(Y)L_n]^+A^- \qquad III$$

où

L représente un ligand neutre,
n est égal à 0, 1 ou 2,
$A^-$ représente un anion tel que $BF_4^-$, $CF_3SO_3^-$, $PF_6^-$, $Cl0_4^-$, $B(C_6H_5)_4^-$ et
Y représente une diphosphine chirale.

3. Procédé selon la revendication 2, caractérisé en ce que la diphosphine chirale est un composé de formule

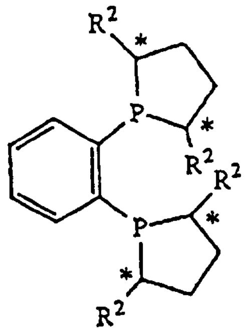

IV

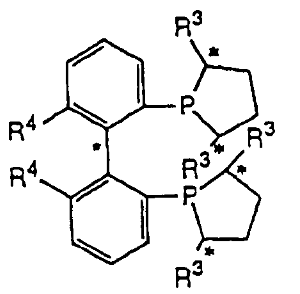

V

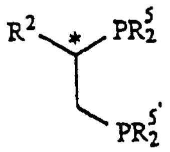

VI

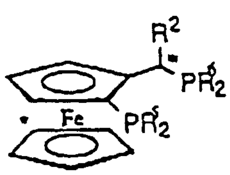

VII

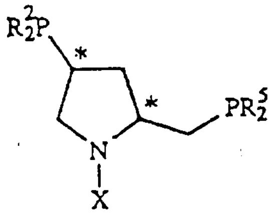

VIII

ou

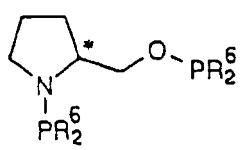

IX

où

X représente -$COR^6$, $COOR^6$, $CONR^3{}_2$, $SO_2R^6$ ou $POR^5{}_2$,

| | |
|---|---|
| $R^2$ | un cycloalkyle ou un alkyle, |
| $R^3$ | l'hydrogène, un cycloalkyle ou un alkyle, |
| $R^4$ | un alkyle inférieur ou un alcoxy inférieur, |
| $R^5$ et $R^{5'}$ | représentent un aryle ou un hétéroaryle, et |
| $R^6$ | représente un aryle, un hétéroaryle, un cycloalkyle ou un alkyle, |
| $R^5$ et $R^{5'}$ | pouvant être identiques ou différents. |

**4.** Procédé selon la revendication 3, caractérisé en ce que la diphosphine chirale est un composé de formule IV ou VII.

**5.** Procédé selon la revendication 3, caractérisé en ce que la diphosphine chirale est un composé de formule IV où $R^2$ représente le méthyle ou l'éthyle.